Europäisches Patentamt

European Patent Office

Office européen des brevets

(11) Veröffentlichungsnummer: **0 111 759**
**A2**

(12) # EUROPÄISCHE PATENTANMELDUNG

(21) Anmeldenummer: **83111481.4**

(22) Anmeldetag: **17.11.83**

(51) Int. Cl.³: **A 61 F 1/00**
**A 61 L 17/00**

(30) Priorität: **11.12.82 DE 3245956**

(43) Veröffentlichungstag der Anmeldung:
**27.06.84 Patentblatt 84/26**

(84) Benannte Vertragsstaaten:
**AT BE CH DE FR GB IT LI NL SE**

(71) Anmelder: **Beiersdorf Aktiengesellschaft**
**Unnastrasse 48**
**D-2000 Hamburg 20(DE)**

(72) Erfinder: **Pietsch, Hanns, Dr.**
**Mittelweg 25**
**D-2000 Hamburg 13(DE)**

(72) Erfinder: **Hohmann, Volker**
**Theodor Fontane Strasse 7a**
**D-2000 Norderstedt(DE)**

(72) Erfinder: **Kluck, Detlef, Dr.**
**Poststrasse 9c**
**D-2150 Buxtehude(DE)**

(54) **Chirurgisches Material.**

(57) Chirurgisches Material auf der Basis von flüssigen monomeren und pulverförmigen polymeren Acryl- und/oder Methacrylsäureestern, Katalysatoren, Beschleunigern und gegebenenfalls üblichen Zusatzstoffen, das durch Vermischen der flüssigen und der pulverförmigen Komponente über einen plastischen in den festen Zustand übergeht und 1 - 15 Gew.-%, bezogen aug das Gesamtgewicht, eine oder mehrere nicht-toxische, flüssige, mit einem Siedepunkt über 100°C, vorzugsweise über 150°C, aliphatische, gesättigte Mono-, Di- oder Tricarbonsäuren mit 1 - 6 Kohlenstoffatomen und gegebenenfalls 1 oder 2 Hydroxygruppen, ganz oder teilweise verestert mit 1-, 2- oder 3-wertigen Alkoholen mit 1 - 4 Kohlenstoffatomen oder flüssige Ethylenglykole oder 1.2- oder 1.3-Propylenglykole mit jeweils 2 bis ca. 30 Glykol-Einheiten enthält.

Beiersdorf Aktiengesellschaft
Hamburg


Chirurgisches Material


Die Erfindung betrifft ein chirurgisches Material auf der Basis von flüssigen monomeren und pulverförmigen polymeren Acryl- und/oder Methacrylsäureestern, das durch den Zusatz von Katalysatoren und Beschleunigern sowie gegebenenfalls üblichen Zusatzstoffen nach dem Vermischen der flüssigen und der pulverförmigen Komponente über einen plastischen in den festen Zustand übergeht. Die Massen eignen sich als sog. "Knochenzemente" insbesondere zum Implantieren von künstlichen Hüft- und Kniegelenken oder dgl. im Knochen.

Knochenzemente auf Basis Methylmethacrylat/Polymethylmethacrylat werden seit vielen Jahren in der Knochenchirurgie verwendet. Sie bestehen aus einer flüssigen und einer festen Komponente, die getrennt gelagert und vor der Anwendung vermischt werden. Die Mischung ist pastös-cremeartig und wird in dieser Form verwendet, dabei erfolgt die Aushärtung nach der Anwendung im Körper. Die feste Komponente besteht üblicherweise aus Polymethylmethacrylat-Pulver, dem Polymerisationsinitiator sowie gegebenenfalls einem Röntgenkontrastmittel wie z.B. Bariumsulfat oder Zirkondioxid.

-2-

Ferner kann das Pulver Antibiotika wie Gentamycin, zur Verstärkung Kurzfasern wie Kohlenstoffasern oder knochenwachstumsfördernde Zusätze wie Calciumphosphat enthalten. Die flüssige Komponente besteht aus monomerem Methylmethacrylat, einem Beschleuniger und gegebenenfalls einem Farbstoff. Die beiden Komponenten werden kurz vor der Verarbeitung im Verhältnis von ca. 2 : 1 vermischt, sind dann etwa 4-6 Minuten verarbeitungsfähig und in 6-15 Minuten ausgehärtet.

Die Vorteile derartiger Knochenzemente sind ihre gute Verträglichkeit mit Gewebematerial, die schnelle Aushärtung sowie die große Festigkeit des ausgehärteten Zements. Diesen Vorteilen stehen als Nachteile jedoch die verhältnismäßig hohen Temperaturen entgegen, die bei der Polymerisation auftreten und zu Schädigungen des umgebenden Gewebes und dadurch zu einer Lockerung des Verbundes führen können.

Ein weiterer Nachteil der bisher für Gelenkoperationen verwendeten "Zemente" auf Methylmethacrylatbasis ist die Abgabe von Restmonomeren. Das monomere Methylmethacrylat gelangt in die Blutbahn und kann zu Fettembolien führen, die cardiozirkulatorische Komplikationen bis zum Herzstillstand nach sich ziehen können.

Diese Nachteile vermeidet ein Knochenzement nach DE-PS 25 52 070, der als flüssige Komponente eine Emulsion aus Methylmethacrylat, Wasser, Emulgator und Beschleuniger enthält. Knochenzemente, die nach diesem Verfahren hergestellt werden, weisen eine geringere maximale Aushärtungstemperatur, geringeren Restmonomergehalt und eine verbesserte Benetzung der Unebenheiten des Knochens auf.

-3-

Neben diesen Vorteilen in der Anwendung weist die Verwendung von Emulsionen jedoch auch Nachteile auf, die vor allem in ihrer Handhabung liegen. Die Emulsionen sind viskose Flüssigkeiten von 200 - 800 MPa·s, die sich nur durch mehrmaliges Schütteln aus der Ampulle entleeren lassen. Dieses Schütteln bedeutet eine lästige, zeitraubende Aktion bei der Operation und es muß sorgfältig darauf geachtet werden, daß die Ampulle vollständig entleert wird und beim Herausschlagen der Emulsion nichts daneben tropft. Außerdem ist eine wäßrige Emulsion bei der Lagerung frostempfindlich.

Aufgabe der Erfindung war es deshalb, ein chirurgisches Material zu entwickeln, welches zwar die in der DE-PS 25 52 070 beschriebenen Vorteile ebenfalls aufweist, jedoch die genannten Nachteile nicht zeigt.

Überraschend wurde gefunden, daß man chirurgische Massen mit verminderter Temperaturentwicklung, vermindertem Gehalt an Restmonomeren, sehr guten Benetzungseigenschaften sowie darüber hinaus mit verbesserter Adhäsion gegenüber Implantatlegierungen erhält, wenn man als flüssige Komponente keine wäßrige Emulsion verwendet, sondern der Masse 1 - 15 Gew.-%, bezogen auf das Gesamtgewicht, eine oder mehrere nicht-toxische, nicht-polymerisierbare, mit dem Methylmethacrylat mischbare organische Flüssigkeiten mit einem Siedepunkt über 100°C, vorzugsweise über 150°C, sowie gegebenenfalls oberflächenaktive Substanzen zufügt.

Bei diesen organischen Flüssigkeiten, die an der Polymerisationsreaktion nicht teilnehmen, da sie keine

-4-

ethylenisch ungesättigten Doppelbindungen enthalten, handelt es sich um flüssige Ester, nämlich aliphatische, gesättigte Mono-, Di- oder Tricarbonsäuren mit 1 - 6 Kohlenstoffatomen und gegebenenfalls 1 oder 2 Hydroxygruppen, die ganz oder teilweise verestert sind mit 1-, 2- oder 3-wertigen Alkoholen mit 1 - 4 Kohlenstoffatomen oder um flüssige Ether, nämlich Ethylenglykole oder 1.2- oder 1.3-Propylenglykole mit jeweils 2 bis ca. 30 Glykol-Einheiten. In der Polymerchemie werden solche Flüssigkeiten auch als Weichmacher bezeichnet. Erstaunlich ist, daß in den angegebenen Mengenverhältnissen keine "Weichmachung" stattfindet und die resultierenden ausgehärteten Massen die gleiche Härte aufweisen wie Polymethylmethacrylat ohne diese Flüssigkeiten.

Von den flüssigen Estern eignen sich in erster Linie:
Glycolmono- und -di- -formiat, -acetat, -propionat, -glycolat, -lactat,
Glycerinmono-, -di- und -tri- -formiat, -acetat, -propionat, -glycolat, -lactat,
Glycolsäure-methyl-, -ethyl-, -propyl- und butylester,
Milchsäure-methyl-, -ethyl-, -propyl- und butylester,
Äpfelsäure-monomethyl- und -dimethylester,
Äpfelsäure-monoethyl- und -diethylester,
Äpfelsäure-monopropyl- und -dipropylester,
Äpfelsäure-monobutyl- und -dibutylester,
Weinsäure-monoethyl- und -dimethylester,
Weinsäure-monoethyl- und -diethylester,
Weinsäure-monopropyl- und -dipropylester,
Weinsäure-monobutyl- und -dibutylester,
Citronensäure-mono-, -di- und -tri-methylester,
Citronensäure-mono-, -di- und -tri-ethylester,

-5-

Citronensäure-mono-, -di- und -tri-propylester,
Citronensäure-mono-, -di- und -tri-butylester.

Den genannten Estern und Ethern, von denen Glycerintriacetat (Triacetin), Citronensäuretriethylester
sowie flüssige Polyethylenglycole, Polypropylenglycole-
(1,2) und Polypropylenglycole-(1,3) mit einem Polymerisationsgrad von n= 5 - 15 besonders bevorzugt sind,
ist gemeinsam, daß sie unpolare und polare Gruppen in
einem Verhältnis von 1:1 bis 3:1 aufweisen, wobei
unter unpolaren Gruppen $-CH_3$, $-CH_2-$, $-CH$ und $-C-$ und
unter polaren Gruppen $-OH$, $-O-$ und $-COOR$ zu verstehen
sind. Diese Tatsache scheint dafür ausschlaggebend zu
sein, daß die Substanzen mit monomerem Methylmethacrylat,
der flüssigen Komponente des Knochenzements, in jedem
Verhältnis mischbar sind und eine klare Lösung bilden
und daß zumindest auch die Ester ein gutes Lösungsmittel
für das Polymethylmethacrylat darstellen. Die Ether sind
ein Quellmittel für das Polymere.

Eine weitere Eigenschaft der genannten Flüssigkeiten
ist ihre sehr gute biologische Verträglichkeit, weshalb
eine Reihe von ihnen auch als Lösungsmittel oder Lösungsvermittler in pharmazeutischen Zubereitungen gebräuchlich sind.

Die Flüssigkeiten haben in dem erfindungsgemäßen
chirurgischen Material u.a. die Funktion, während der
Anrührphase der plastischen Masse eine cremige Konsistenz zu geben und die Viskosität zu reduzieren. Dabei
zeigte es sich, daß sie bezüglich der Eigenschaften
der mit ihnen zubereiteten Knochenzemente zwei Gruppen
bilden:

-6-

1. Die Gruppe der Ether, d.h. Ethylen- und Propylenglykole, und der Ester von Glykol und Glyzerin mit Carbonsäuren, welche keine Hydroxylgruppen aufweisen, ergeben Knochenzemente, die denen der DE-PS 25 52 070 vergleichbar sind.

2. Die Gruppe der Ester von Hydroxycarbonsäuren, wie Glycolsäure, Milchsäure, Äpfelsäure, Weinsäure oder Citronensäure, ergeben Knochenzemente, die sich von denen der ersten Gruppe deutlich dadurch unterscheiden, daß die diese Verbindungen enthaltenden Mischungen im plastischen Zustand eine ausgeprägte Klebrigkeit aufweisen.

Die Flüssigkeitsmischungen aus Methylmethacrylat, der beschriebenen Flüssigkeit, Beschleuniger und gegebenenfalls 0 - 5% eines oberflächenaktiven Mittels werden vor der Verwendung mit einer Pulvermischung verrührt. Diese Pulvermischung besteht aus Polymethylmethacrylat-Perlpolymerisat, Polymerisationsinitiator und gegebenenfalls einem Röntgenkontrastmittel. Diese Pulvermischung ist nicht Gegenstand der vorliegenden Erfindung. Das Verhältnis von Pulver zu flüssiger Phase beträgt 1 - 3, vorzugsweise 1,5 - 2,5. Die in der flüssigen Komponente enthaltene erfindungsgemäße organische Flüssigkeit ist in der nach dem Vermischen von Pulver und flüssiger Komponente entstandenen Masse in einem Anteil von 1 bis 15% enthalten.

Üblicherweise werden plastische Knochenzementmischungen einige Zeit nach dem Zusammengeben der flüssigen und pulverförmigen Komponente klebfrei. Sie können sodann in die Hand genommen und verformt werden, ohne daß Rückstände an der Hand oder dem Handschuh

-7-

zurückbleiben.

Die Zeit vom Vermischen der beiden Komponenten bis zur Klebfreiheit wird nach DIN-ISO 5833 "Anteigzeit" genannt. Erfindungsgemäße Knochenzemente mit einem Anteil von Verbindungen der erstgenannten Gruppe erreichen diesen klebfreien Zustand vor dem Einbringen in den Knochen. Erfindungsgemäße Knochenzemente mit einem Anteil von Verbindungen der zweiten Gruppe erreichen diesen Zustand der Klebfreiheit nicht vor dem Erhärten. Sie lassen sich vorzugsweise mit einer sogenannten Knochenzementspritze applizieren und haben eine ausgezeichnete Adhäsion zu polaren Materialien, wie metallische Prothesen, und Knochen.

Wie bereits beschrieben, werden die flüssigen Ether und Ester vorzugsweise zusammen mit dem monomeren Methylmethacrylat und dem Beschleuniger in einer Mischung als "flüssige Komponente" verwendet. Es ist aber auch möglich, diese für sich getrennt, z.B. als eine weitere Komponente, mit dem Pulver anzurühren. Für die Eigenschaften des ausgehärteten Knochenzements ist die Art der Zugabe nicht wesentlich. Jedoch können die Verarbeitungseigenschaften dadurch beeinflußt werden.

In der bevorzugten Ausführungsform des chirurgischen Materials, bei welchem die Ether und Ester dem flüssigen Monomeren zugemischt sind, besteht die flüssige Phase im wesentlichen aus

94,5 - 62 Gew.-% monomerem Methylmethacrylat, gegebenenfalls mit geringen Anteilen modifizierender anderer Acrylat-Derivate,

5 - 30 Gew.-%, vorzugsweise 15 - 20 Gew.-%, der genannten flüssigen Ester und/oder Ether

0,5 - 3 Gew.-% Beschleuniger und

0 - 5 Gew.-% oberflächenaktiven Substanzen.

Dabei ist der Methacrylsäuremethylester zur besseren Lagerfähigkeit mit 50 - 100 ppm Hydrochinon oder Hydrochinonmonomethylester stabilisiert.

Als Beschleuniger für die spätere Polymerisationsreaktion verwendet man üblicherweise N,N-Dimethyl-p-toluidin, N,N-Dimethylanilin, N,N-Dimethylxylidine oder N,N-Dimethyl-anisidine bzw. deren N-Monomethylderivate, vorzugsweise das Erstgenannte.

Es hat sich gezeigt, daß derartige Mischungen eine gute Benetzung der unebenen Knochenoberfläche ergeben; diese und die Fähigkeit, auch die feinsten Rillen auszufüllen, läßt sich jedoch noch verbessern, wenn man einen gewissen Anteil an oberflächenaktiven (grenzflächenaktiven) Substanzen hinzugibt. Hierfür eignen sich vorzugsweise nichtionische und kationenaktive Verbindungen wie z.B. Fettsäureester des Sorbitans, des ethoxylierten Sorbitans, des Sorbitols, ethoxylierte Fettsäuren, ethoxylierte aliphatische Alkohole, partielle Fettsäureester des Glycerins, ethoxylierte partielle Fettsäureester des Glycerins, Alkyldimethyl-benzyl-ammoniumchlorid, Alkylammoniumbenzoat, -lactat, Cetylpyridiniumchlorid, Dodecyl-di(ß-oxyethyl)benzylammoniumchlorid oder Sojatrimethylammoniumchlorid, die einzeln oder im Gemisch eingesetzt werden können.

Außerdem kann die flüssige Phase gegebenenfalls noch geringe Mengen anderer Hilfsstoffe, wie z.B. Färbemittel, enthalten.

Die pulverförmige, feste Komponente des chirurgischen Materials besteht in an sich bekannter Weise in erster Linie aus dem sehr feinkörnigen Polymethylmethacrylat (mittlerer Korndurchmesser bis zu 200 /um) und gegebenenfalls geringen Anteilen an modifizierenden anderen Polyacrylsäureesterderivaten oder Copolymeren davon, wie z.B. Methylmethacrylat/Ethylacrylat-Copolymer, Methylmethacrylat/Butylmethacrylat-, Methylmethacrylat/ Methylacrylat- oder Methylmethacrylat/Butylacrylat- Copolymer, dem Polymerisationsinitiator - üblicherweise etwa 1 - 2% Dibenzoylperoxid - und einem Röntgenkontrastmittel wie beispielsweise Zirkondioxid, Cerdioxid, Thoriumdioxid, Barium- oder Calciumsulfat. Außerdem kann diese Komponente noch andere Zusätze wie Antibiotika, bioabbaubare Substanzen wie Tricalciumphosphat und Kollagen, bioaktive Glaskeramik oder verstärkende Kurzfasern aus beispielsweise Kohlenstoff, Polyestern, Polyvinylalkohol oder Polyamid enthalten.

Bei der Anwendung des chirurgischen Materials, vielfach z.B. bei der Implantation eines künstlichen Hüftgelenks, werden die getrennt voneinander aufbewahrten, sterilen Komponenten in den jeweils erforderlichen Mengen miteinander zu einer gleichmäßigen Paste verrührt und müssen dann vergleichsweise rasch verarbeitet werden, da die Polymerisationsreaktion alsbald unter Selbsterwärmung einsetzt und die Massen nach kurzer Zeit durchhärten.

Das Verhältnis von Flüssigkeit zu Pulver beträgt etwa 0,4 bis 0,75, wobei man, um den Monomeranteil gering zu halten, vorzugsweise an der unteren Grenze bleibt; andererseits sollte der Anteil der Flüssigkeit nicht zu niedrig sein, weil sonst die Viskosität der

-10-

angerührten Masse zu hoch ist und sich die Masse deswegen weniger gut verrühren läßt.

Um die Eigenschaften der erfindungsgemäßen chirurgischen Mischungen darzustellen, wurden die folgenden Beispiele durchgeführt. Die Messungen der Eigenschaften erfolgten nach der DIN-ISO-Norm 5833 für sogenannte Knochenzemente. Die Restmonomergehalte wurden durch Head-Space-Analyse gaschromatographisch ermittelt.

Beispiel 1

Es wurde ein Flüssigkeitsgemisch hergestellt bestehend aus 79 Gew.-% Methacrylsäuremethylester, 20 Gew.-% Triacetin sowie 1 Gew.-% N,N-Dimethyl-p-toluidin. Die Mischung ist eine klare homogene Flüssigkeit bei Raumtemperatur und läuft in wenigen Sekunden aus einer Ampulle aus, ohne daß Schütteln erforderlich ist.

18g dieser Flüssigkeitsmischung wurden vermischt mit 42g einer Pulvermischung bestehend aus 88,6 Gew.-% Polymethylmethacrylat-Pulver, 10 Gew.-% Zirkondioxidpulver sowie 1,4 Gew.-% Dibenzoylperoxid.

An dieser chirurgischen Masse wurden folgende Werte nach DIN-ISO 5833 gemessen:

| | | |
|---|---|---|
| Aushärtezeit bei 24$^{\circ}$C | : | 11' 30" |
| Maximale Aushärtetemperatur: | | 49,5$^{\circ}$C |
| Intrusion | : | 8 mm |
| Druckfestigkeit | : | 87,5 MPa |
| Eindringtiefe | : | 0,166 mm |

-11-

Rückstellung                    : 73,5%

Restmonomer-Gehalt              : 0,15%


Beispiele 2 - 9


Es wurden folgende Flüssigkeitsmischungen aus 79 Gew.-% Methacrylsäuremethylester, 1 Gew.-% N,N-Dimethyl-p-toluidin sowie je 20 Gew.-%

Beispiel 2 :    Glycerindiacetat

"        3 :    Glycoldiacetat

"        4 :    Polyethylenglycol 400 (n = 9)

"        5 :    Polyethylenglycol 420 (n = 9,5)

"        6 :    Polypropylenglycol 620 (n = 10,7)

"        7 :    Polypropylenglycol 1020 (n = 17,6)

"        8 :    L(+)-Weinsäurediethylester

"        9 :    Citronensäuretriethylester

hergestellt.

Von diesen Flüssigkeitsmischungen, die völlig klar und homogen waren, wurden je 18g mit 42g der in Beispiel 1 beschriebenen Pulvermischung verrührt und die Eigenschaften nach DIN-ISO 5833 gemessen sowie die Restmonomergehalte im ausgehärteten Knochenzement bestimmt:

-12-

| Beisp. | Maximale Aushärte- temperatur ($^\circ$C) | Intru- sion (mm) | Druck- festig- keit (MPa) | Ein- dring- tiefe (mm) | Rück- stellung ( % ) |
|---|---|---|---|---|---|
| 2 | 52,5$^\circ$C | 7,5 | 80,7 | 0,184 | 71,7 |
| 3 | 49$^\circ$C | 7 | 72,5 | 0,185 | 62,2 |
| 4 | 48$^\circ$C | 3,5 | 74,5 | 0,160 | 61,4 |
| 5 | 49$^\circ$C | 6,4 | 75 | 0,155 | 67,7 |
| 6 | 48$^\circ$C | 8,5 | 70,5 | 0,179 | 70,4 |
| 7 | 44,5$^\circ$C | 5,3 | 81,4 | 0,150 | 67,3 |
| 8 | 49,0$^\circ$C | x) | 101,3 | 0,145 | 77,6 |
| 9 | 47$^\circ$C | x) | 94,2 | 0,198 | 83 |

x) Nicht meßbar, da der Zustand der Klebfreiheit nicht erreicht wurde.

## Beispiele 10 - 14

Folgende Flüssigkeitsmischungen wurden mit nach Art und Menge unterschiedlichen erfindungsgemäßen orga- nischen Flüssigkeiten hergestellt:

| Beisp. | MMA[1] | DMPT[2] | Flüssigkeit | |
|---|---|---|---|---|
| 10 | 93 Gew.-% | 2 Gew.-% | 5 Gew.-% | Milchsäure- ethylester |
| 11 | 88 " | 2 " | 10 " | Milchsäure- ethylester |
| 12 | 73 " | 2 " | 25 " | Milchsäure- ethylester |
| 13 | 78 " | 2 " | 20 " | Citronen- säure- triethylester |
| 14 | 73 " | 2 " | 25 " | Citronen- säure- triethylester |

[1] Methylmethacrylat
[2] N,N-Dimethyl-p-toluidin

-13-

Von diesen Mischungen, die völlig klar und homogen waren, wurden je 18g mit 42g der im Beispiel 1 beschriebenen Pulvermischung verrührt und die Eigenschaften nach DIN-ISO 5833 gemessen sowie die Restmonomergehalte in der ausgehärteten Masse bestimmt. Es wurden folgende Ergebnisse gefunden:

| Beisp. | Maximale Aushärtetemperatur ($^O$C) | Druckfestigkeit (MPa) | Eindringtiefe (mm) | Rückstellung ( % ) |
|---|---|---|---|---|
| 10 | 51 | 114,2 | 0,153 | 75,2 |
| 11 | 47 | 107,1 | 0,149 | 75,2 |
| 12 | 39,5 | 70,3 | 0,207 | 60,2 |
| 13 | 41,5 | 100 | 0,161 | 72,0 |
| 14 | 39 | 80,5 | 0,198 | 66,6 |

Die Massen blieben während der Verarbeitungszeit alle klebrig.

Beispiele 15 und 16

Es wurden folgende Flüssigkeitsgemische hergestellt:

| | Beispiel 15 | Beispiel 16 |
|---|---|---|
| Methylmethacrylat | 77 Gew.-% | 76 Gew.-% |
| N,N-Dimethyl-p-toluidin | 1,5 " | 1,5 " |
| Citronensäuretriethylester | 20 " | 20 " |
| Polyoxyethylen-(5)-sorbitan-monooleat | 1,5 " | 2,5 " |

-14-

Von diesen klaren und homogenen Mischungen wurden je 18g mit je 42g Pulvermischung gemäß Beispiel 1 vermischt und die Eigenschaften nach DIN-ISO 5833 gemessen:

| Beisp. | Maximale Aushärte- temperatur ($^{o}$C) | Druck- festig- keit (MPa) | Eindring- tiefe (mm) | Rück- stellung ( % ) |
|---|---|---|---|---|
| 15 | 45 | 96 | 0,172 | 78 |
| 16 | 42,5 | 92 | 0,185 | 75 |

Von verschiedenen Mischungen wurden nach der Aushärtung der Restmonomeren-Gehalt gaschromatographisch nach der Head-Space-Methode ermittelt und folgende Werte gemessen:

| Beispiel | Restmonomerengehalt |
|---|---|
| 1 | 0,15 % |
| 5 | 0,15 % |
| 8 | 0,50 % |
| 12 | 0,35 % |
| 16 | 0,20 % |

Das neue chirurgische Material mit dem erfindungsgemäßen Zusatz der speziellen organischen Flüssigkeiten weist alle vorteilhaften Eigenschaften auf, welche an einen hochqualitativen Knochenzement gestellt werden, wie ein Vergleich seiner Meßdaten für die Druckfestigkeit, welche mehr als 70 MPa betragen soll, für die Eindringtiefe, die nicht mehr als 0,2 mm groß sein soll, und die Rückstellung, die mehr als 60% ergeben soll, mit den marktgängigen Produkten zeigte. Darüber hinaus besitzt es eine im Vergleich zu diesen verringerte maximale

-15-

Aushärtungstemperatur von 50°C oder kleiner, ferner
- bedingt durch den reduzierten Monomerengehalt - einen
geringeren Restmonomerengehalt sowie deutlich höhere
Werte der Intrusion, die ein Maß dafür ist, wie gut die
Masse im noch plastischen Zustand feine Details auszufüllen vermag, und ist damit einem Knochenzement gemäß
der DE-PS 25 52 070 ebenbürtig ohne die beschriebenen
Nachteile einer Emulsion zu zeigen.

0111759

– 16 –

Patentansprüche

1. Chirurgisches Material auf der Basis von flüssigen monomeren und pulverförmigen polymeren Acryl- und/ oder Methacrylsäureestern, Katalysatoren, Beschleunigern und gegebenenfalls üblichen Zusatzstoffen, das durch Vermischen der flüssigen und der pulverförmigen Komponente über einen plastischen in den festen Zustand übergeht, dadurch gekennzeichnet, daß es 1 – 15 Gew.-%, bezogen auf das Gesamtgewicht, eine oder mehrere nicht-toxische, flüssige, mit einem Siedepunkt über 100°C, vorzugsweise über 150°C, aliphatische, gesättigte Mono-, Di- oder Tricarbonsäuren mit 1 – 6 Kohlenstoffatomen und gegebenenfalls 1 oder 2 Hydroxygruppen, ganz oder teilweise verestert mit 1-, 2- oder 3-wertigen Alkoholen mit 1 – 4 Kohlenstoffatomen oder flüssige Ethylenglykole oder 1.2- oder 1.3-Propylenglykole mit jeweils 2 bis ca. 30 Glykol-Einheiten enthält.

2. Chirurgisches Material gemäß Anspruch 1, dadurch gekennzeichnet, daß die flüssigen Ester und Ether ein Verhältnis von unpolaren zu polaren Gruppen von 1 : 1 bis 3 : 1 aufweisen, wobei als unpolare Gruppen $-CH_3$, $-CH_2-$, $-\overset{|}{C}H$ und $-\overset{|}{\underset{|}{C}}-$ und als polare Gruppen $-OH$, $-O-$ und $-COOR$ gelten.

3. Chirurgisches Material gemäß Anspruch 1 und 2, dadurch gekennzeichnet, daß der flüssige Ester oder Ether Glycerintriacetat, Citronensäuretriethylester, Polyethylenglycol oder Polypropylenglycol-(1,2) und (1,3) mit Polymerisationsgraden von 2 bis 30 ist.

-17-

4. Chirurgisches Material gemäß Anspruch 1, 2 oder 3, dadurch gekennzeichnet, daß der flüssige Ester oder Ether in der flüssigen Komponente enthalten ist.

5. Chirurgisches Material gemäß Anspruch 4, dadurch gekennzeichnet, daß der flüssige Ester oder Ether in der flüssigen Komponente mit einem Anteil von 5 - 30 Gew.-%, vorzugsweise 15 - 20 Gew.-%, enthalten ist.

6. Chirurgisches Material gemäß einem oder mehreren der Ansprüche 1 bis 5, dadurch gekennzeichnet, daß die flüssige Komponente bis zu 5% einer oberflächenaktiven Substanz enthält.

7. Chirurgisches Material gemäß Anspruch 1 bis 6, dadurch gekennzeichnet, daß das Verhältnis von Pulver zu flüssiger Komponente 1 bis 3, vorzugsweise 1,5 bis 2,5 beträgt.

8. Verwendung der chirurgischen Masse gemäß einem oder mehreren der Ansprüche 1 bis 7 als Knochenzement.

9. Verwendung von nicht-toxischen, flüssigen, mit einem Siedepunkt über 100°C, vorzugsweise über 150°C, aliphatischen, gesättigten Mono-, Di- oder Tricarbonsäuren mit 1 - 6 Kohlenstoffatomen und gegebenenfalls 1 oder 2 Hydroxygruppen, ganz oder teilweise verestert mit 1-, 2- oder 3-wertigen Alkoholen mit 1 - 4 Kohlenstoffatomen oder flüssigen Ethylenglykolen oder 1.2- oder 1.3-Propylenglykolen mit jeweils 2 bis ca. 30 Glykol-Einheiten in Mengen von 1-15 Gew.-%, bezogen auf das Gesamtgewicht, als Zusatz zu chirur-

-18-

gischem Material auf der Basis von flüssigen monomeren und pulverförmigen polymeren Acryl- und/oder Methacrylsäureestern, Katalysatoren, Beschleunigern und gegebenenfalls üblichen Zusatzstoffen, das durch Vermischen der flüssigen und der pulverförmigen Komponente über einen plastischen in den festen Zustand übergeht.